# NEUE EUROPÄISCHE PATENTSCHRIFT

(11) **EP 0 919 619 B2**
(45) Veröffentlichungstag und Bekanntmachung des Hinweises auf die Entscheidung über den Einspruch: **19.06.2013**
(45) Hinweis auf die Patenterteilung: 01.03.2006
(21) Anmeldenummer: 98122807.5
(22) Anmeldetag: 01.12.1998
(51) Int. Cl.: C12N 15/12, C12N 15/65, C12N 15/67, C12N 15/85, C12N 15/90, C12N 5/10, C07K 14/505, C12Q 1/68

(54) **Optimierung von Zellen für die endogene Genaktivierung**
Optimizing cells for endogenous gene activation
Optimalisation de cellules pour l'activation de l'expression de gènes endogènes

(30) Priorität: 01.12.1997 EP 97121075
(43) Veröffentlichungstag der Anmeldung: 02.06.1999
(60) Teilanmeldung: 99112607.9
(73) Patentinhaber: Roche Diagnostics GmbH, 68305 Mannheim (DE)
(72) Erfinder: Honold, Konrad, 82377 Penzberg (DE); Holtschke, Thomas, 81369 München (DE); Stern, Anne, 82377 Penzberg (DE)
(74) Vertreter: Weiss, Wolfgang

(56) Entgegenhaltungen:
- EP-A- 0 220 009
- EP-A- 0 747 485
- WO-A-90/11354
- WO-A-91/09955
- WO-A-92/15694
- WO-A-94/17176
- WO-A-96/29411
- WO-A-96/30498
- US-A- 4 959 317
- Z.-W. LI ET AL.: "Generation of mice with a 200-kb amyloid precursor protein gene deletion by Cre recombinase-mediated site specific recombination in embryonic stem cells" PROC. NATL. ACAD. SCI., Bd. 93, Juni 1996, Seiten 6158-6162, XP002095251 NATL. ACAD. SCI.,WASHINGTON,DC,US;
- BAUBONIS W ET AL: "GENOMIC TARGETING WITH PURIFIED CRE RECOMBINASE" NUCLEIC ACIDS RESEARCH, Bd. 21, Nr. 9, 11. Mai 1993, Seiten 2025-2029, XP000615551
- METZGER D ET AL: "CONDITIONAL SITE-SPECIFIC RECOMBINATION IN MAMMALIAN CELLS USING A LIGAND-DEPENDENT CHIMERIC CRE RECOMBINASE" PROCEEDINGS OF THE NATIONAL ACADEMY OF SCIENCES OF USA, Bd. 92, Nr. 15, 18. Juli 1995, Seiten 6991-6995, XP000615550
- MERRIHEW R V ET AL: "EFFICIENT MODIFICATION OF THE APRT GENE BY FLP/FRT SITE-SPECIFIC TARGETING" SOMATIC CELL AND MOLECULAR GENETICS, Bd. 21, Nr. 5, September 1995, Seiten 299-307, XP000614587
- FUKUSHIGE S ET AL: "GENOMIC TARGETING WITH A POSITIVE-SELECTION LOX INTEGRATION VECTOR ALLOWS HIGHLY REPRODUCIBLE GENE EXPRESSION IN MAMMALIAN CELLS" PROCEEDINGS OF THE NATIONAL ACADEMY OF SCIENCES OF USA, Bd. 89, Nr. 17, 1. September 1992, Seiten 7905-7909, XP000615553

## Beschreibung

Die Erfindung betrifft Verfahren zur Optimierung der Genexpression in Zellen. Ein erster Aspekt betrifft ein Verfahren zum Verändern der Expression eines in einer eukaryontischen Zelle endogen vorliegenden Zielgens durch Einführen einer heterologen Expressionskontrollsequenz und gegebenenfalls zusätzlich eines Amplifikationsgens in das Genom der Zelle mittels homologer Rekombination, sowie das durch eine ortsspezifische Rekombinase vermittelte Herausschneiden der insertierten Fremd-DNA und ihr Ersetzen durch weitere heterologe Expressionskontrollsequenzen oder/und Amplifikationsgenen.

Die Genexpression in einer Zelle kann konstitutiv, beispielsweise bei sogenannten Housekeeping-Genen, oder reguliert erfolgen. Die regulierte Expression ist insbesondere für Gene notwendig, die nur in einem bestimmten Entwicklungsstadium der Zelle oder bei einer Änderung der Umweltbedingungen exprimiert werden müssen.

Die Expression wird auf der Transkriptionsebene durch den operativ mit der kodierenden Nukleinsäuresequenz verbundenen Promotor reguliert, dessen Aktivität durch Repressoren und Aktivatoren gesteuert werden kann. Eine Bindung von Repressoren bzw. Aktivatoren an nichtkodierende Nukleinsäuresequenzen des Gens kann eine Verminderung bzw. Erhöhung der Aktiviät des Promotors bewirken (L. Stryer, Biochemie, Kapitel 12, Spektrum der Wissenschaft, Verlagsgesellschaft, Heidelberg, 1990). Die Menge der in einer Zelle enthaltenen Repressoren bzw. Aktivatoren wird wiederum durch Faktoren, wie beispielsweise Umweltbedingungen, reguliert. Ein Beispiel für Aktivatoren sind die Hypoxia-Inducible-Factoren (HIF), die durch vermindertes O₂-Angebot induziert werden und zu einer erhöhten Expression des Erythropoietingens führt (Blanchard K.L. et al., Hypoxic induction of the human erythropoietin gene: Cooperation between the promotor and enhancer, each of which contains steroid receptor response elements, (1992), Mol.Cell.Biol. 12, 5373-5385; Wang G.L. and Semenza G.L., Characterization of hypoxia-inducible factor 1 and regulation of DNA binding activity by hypoxia, (1993), J.Biol.Chem., 268, 21513-21518; Wang G.L. et al., Hypoxia-inducible factor 1 is a basie-helix-loop-helix-PAS heterodimer regulated by cellular O2 tension, (1995), Proc.Natl.Acad.Sci. USA, 92, 5510-5514).

Desweiteren ist die Menge eines exprimierten Proteins von der Stabilität der mRNA abhängig. Im 3'-seitigen Bereich einer mRNA sind Erkennungssequenzen für mRNA abbauende Enzyme lokalisiert, die die Stabilität der mRNA und somit die Expressionshöhe beeinflussen (Shaw G. and Kamen R., A Conserved AU Sequence from the 3'Untranslated Region of GM-CSF mRNA Mediates Selective mRNA Degradation, Cell (1986), 659-667). Die Halbwertszeit der mRNA korreliert dabei mit der Menge exprimierten Proteins. Eine dritte Ebene der Expressionsregulation ist die Translation.

Die Expression eines Gens unterliegt somit komplexen Regulationsmechanismen, die im Einzelfall sehr unterschiedlich sein können.

Proteine können mit Hilfe der rekombinanten DNA-Technologie gewonnen werden, welche die Kenntnisse der Expressionsregulation nutzt (Sambrook et al., 1989 Molecular Cloning, A Laboratory Manual, Cold Spring Harbor). Hierzu werden Vektoren verwendet, die eine das entsprechende Protein kodierende Nukleinsäuresequenz unter Kontrolle eines geeigneten Promotors enthalten, sowie weitere für die Expression des Proteins und zur Replikation des Vektors notwendige Sequenzen. Der Vektor wird dann mittels bekannter Verfahren in eine Wirtszelle eingebracht, die Zelle wird kultiviert und das rekombinante Protein kann aus der Zelle oder dem Kulturmedium gewonnen werden.

Als Wirtszelle können prokaryontische oder eukaryontische Zellen verwendet werden. Prokaryontische Zellen, insbesondere E.coli-Zellen, sind in ihrer Handhabung unproblematisch, weisen aber bei einer rekombinanten Expression von eukaryontischen Proteinen eine Reihe von Nachteilen auf.

Prokaryonten und Eukaryonten unterscheiden sich im Expressionsprozessierungsweg, in den Zellmilieu-Bedingungen sowie in den bei der Proteinprozessierung beteiligten Chaperons. Deshalb können in einem in Prokaryonten hergestellten eukaryontischen Protein entscheidende Unterschiede im Vergleich zu dem entsprechenden nativen Protein auftreten. Beispielsweise kann das Proteinfaltungsmuster und die Aktivität des Proteins verändert sein. Auch werden Proteine in einer prokaryontischen Wirtszelle in der Regel nicht glycosyliert. Ein korrektes Glycosylierungsmuster stellt aber in vielen Fällen, beispielsweise bei der Herstellung von Proteinen für eine pharmazeutische Formulierung, ein entscheidendes Merkmal für die Wirksamkeit und Verträglichkeit dar.

Glycosylierte Proteine werden deshalb mittels eukaryontischer Wirtszellen oder Zellinien, beispielsweise CHO (Chinese Hamster Ovary) Zellen, hergestellt. Trotz der Verwendung eukaryontischer Zellen können aufgrund von Speziesunterschieden, beispielsweise bei der Expression eines humanen Proteines in nichthumanen Zellen, Veränderungen in dem rekombinant hergestellten Protein auftreten, wodurch dieses für viele Anwendungen unbrauchbar wird.

Zur rekombinanten Herstellung von Proteinen werden Wirtszellen transient oder stabil mit Expressionsvektoren transfiziert, wobei insbesondere bei großtechnischen Herstellungsverfahren stabil transfizierte Zellen verwendet werden.

Die unspezifische, zufällige Integration der Expressionsvektorsequenzen in das Genom der Wirtszelle kann zu Zellen mit geringer Produktionsleistung oder instabilen Eigenschaften der Zellen führen. Beispielsweise kann im Laufe des Produktionsprozesses die Produktionsleistung sinken oder die Fähigkeit der Zellen das rekombinante Protein zu exprimieren geht ganz verloren.

Ein Verfahren zur Erhöhung der Genexpression stellt die Genamplifikation dar, bei der eine für ein Protein kodierende Nukleinsäuresequenz mit einem Amplifikationsgen gekoppelt wird. Durch einen Selektionsschritt erreicht man eine Vervielfältigung beider Sequenzen, die zu einer erhöhten Expression führt (Schimke R.T. (Ed.) (1982), Gene amplifikation, Cold Spring Harbor Lab., Cold Spring Harbor, NY).

Als Amplifikationsgen kann beispielsweise eine für eine Dihydrofolatreduktase (DHFR) kodierende Nukleinsäure verwendet werden (Kaufmann R.J., Sharp P.A. (1982), Amplifikation and expression of sequences cotransfected with a modular dihydrofolate reductase complementary DNA gene, J. Mol. Biol. 159:601ff).

Durch einen mit Methotrexat durchgeführten Selektionsschritt erhält man Zellen, die gegenüber Methotrexat resistent sind und in ihrem Genom die für eine DHFRkodierende und mit ihr gekoppelte Nukleinsäuresequenz in 20 bis 50-facher Amplifikation enthalten (R. Knippers, 1982, Molekulare Genetik, Thieme, Stuttgart).

Ein derartiges Genamplifikationsverfahren wird am effektivsten mit einer DHFRnegativen Zelle durchgeführt. JP-62265992 beschreibt z.B. eine humane DHFRnegative Zelle. Ein Hinweis auf eine ortsspezifische Integration eines Expressionsvektors mittels homologer Rekombination und Amplifikation dieser Sequenzen in dieser Zelle findet sich darin jedoch nicht.

Auch bei der Durchführung eines Genamplifikationsverfahrens können aufgrund zufälliger Integration des Expressionsvektors in das Genom der Zelle die oben dargestellten Nachteile, wie beispielsweise Instabilität der Zellen, auftreten.

Lediglich bei einer ortsspezifischen Integration von Fremd-DNA an einem ausgewählten Genlocus durch homologe Rekombination, die zu einer endogenen Genaktivierung führt, können die beschriebenen Nachteile vermieden werden. Entsprechende Verfahren sind bekannt und werden als Gentargeting bezeichnet (WO 90/11354; WO 91/09955). Dabei wird die Zelle mit einem Vektor transfiziert, der ein positives Selektionsmarkergen enthält, flankiert von Nukleinsäuresequenzen, die homolog zu Sequenzen eines Genlocus sind, an dem der Vektor in das Genom der Zelle integriert werden soll. Zwischen den homologen Nukleinsäuresequenzen befindet sich weiterhin eine heterologe Expressionskontrollsequenz, um die Expression des Zielgens in der Zelle zu erhöhen, und gegebenenfalls ein Amplifikationsgen, um die Kopienzahl des Zielgens zu vergrößern.

Ein Nachteil bisher bekannter Gentargeting-Verfahren besteht darin, daß die Herstellung von Zellen mit Eigenschaften, die die Herstellung eines gewünschten Proteins in einer für kommerzielle Zwecke ausreichenden Menge und Qualität ermöglichen, oft mit sehr hohem Aufwand verbunden ist. Insbesondere die Auswahl von optimalen Expressionskontrollsequenzen oder/und Amplifikationsgenen für die Expression eines gewünschten Zielproteins erfordert oft eine beträchtliche Anzahl von Versuchsreihen zur homologen Rekombination, die aufgrund der aufwendigen Prozedur zur Isolierung von Klonen, in denen das gewünschte Rekombinationsereignis stattgefunden hat, mitsehr hohem Aufwand verbunden sind.

Die homologe Rekombination kann auch verwendet werden, um die Expression bestimmter Gene in einer Zelle auszuschalten und Protein-Funktionsstudien durchzuführen. Hierzu werden Knockout-Mäuse erzeugt, indem das für ein zu untersuchendes Protein kodierendes Gen in embryonalen Stammzellen durch homologe Rekombination ausgeschaltet wird. Nach Durchführung weiterer Verfahrensschritte werden Mäuse erhalten, die aufgrund der Inaktivierung beider Allele dieses Gens vom Beginn ihrer Entwicklung kein funktionelles Protein exprimieren können (Thomas K.R., Capecchi M.R., (1987), Site-directed mutagenesis by gene targeting in mouse embryo-derived stem cells, Cell 51: 503-512).

Um ein bestimmtes Gen gewebe- und zeitspezifisch auszuschalten und zu untersuchen, kann das Cre-Lox-System eingesetzt werden. Hierbei wird ein von zwei loxP-Sequenzen flankiertes Nukleinsäurefragment durch homologe Rekombination in das Genom einer Zelle eingebracht und kann anschließend durch eine in der Zelle exprimierte Cre-Rekombinase wieder aus dem Genom herausgeschnitten werden (Sauer B, Henderson N (1989): Site-specific DNA recombination at loxP sites placed into the genome of mammalian cells. Nuc Acid Res 17:147-161; Sauer B., Henderson N. (1990), Targeted insertion of exogenous DNA into the eukaryotic genome by the Cre recombinase, New Biol. 5:441-449). Ein Hinweis auf eine Verwendung des Cre-lox-Systems oder eines anderen ortsspezifischen Rekombinasesystems für die ortspezifische Integration von Expessionskontrollsequenzen oder Amplifikationsgenen in das Genom von eukaryontischen Zellen zur Veränderung der endogenen Genexpression findet sich im Stand der Technik nicht.

Die der vorliegenden Erfindung zugrunde liegende Aufgabe bestand darin, ein neues Verfahren zur Optimierung der endogenen Genaktivierung durch homologe Rekombination bereitszustellen, bei dem die Nachteile des Standes der Technik zumindest teilweise beseitigt werden.

Diese Aufgabe wird gelöst durch Bereitstellung neuer Verfahren und Vektorkonstrukte, die eine Optimierung der Expressionsleistung von Genen in eukaryontischen Zellen ganz erheblich erleichtern. Ein erster Aspekt der Erfindung betrifft ein Verfahren zum Verändern der Expression eines in einer eukaryontischen Zelle endogen vorliegenden gens, das dadurch gekennzeichnet ist, daß
(a) die Zelle transfiziert wird mit einem ersten Vektor, umfassend
   (i) eine heterologe Expressionskontrollsequenz, um die Expression des Zielgens in der Zelle zu erhöhen, und gegebenenfalls zusätzlich ein Amplifikationsgen,
   (ii) ein positives Selektionsmarkergen,
   (iii) mindestens zwei die Sequenzen (i) und (ii) flankierende Zielsequenzen für eine ortsspezifische Rekombinase,
   (iv) die Sequenzen (i), (ii) und (iii) flankierende DNA-Sequenzen, die homolog zu einem Nukleinsäureabschnitt im Genom der Zelle sind, um eine homologe Rekombination zu erlauben,
(b) die transfizierte Zelle unter Bedingungen kultiviert wird, unter denen eine homologe Rekombination des Vektors erfolgt, und
(c) die gemäß Schritt (b) erhaltene Zelle gewonnen wird, die das endogene Gen in operativer Verknüpfung mit der heterologen Expressionskontrollsequenz aufweist, wobei diese Sequenzen flankiert sind von den Zielsequenzen für die ortsspezifische Rekombinase.

Mit dem erfindungsgemäßen Verfahren wird eine Zelle bereitgestellt, die ein endogenes Gen in operativer Verknüpfung mit einer heterologen Expressionskontrollsequenz, um die Expression des Zielgens in der Zelle zu erhöhen, und gegebenenfalls zusätzlich einem Amplifikationsgen aufweist, wobei diese Sequenzen flankiert sind von Zielsequenzen für eine ortsspezifische Rekombinase, z.B. der Cre-Rekombinase. Diese Zelle eignet sich hervorragend für Untersuchungen zur Optimierung der Expression des Zielgens, da aufgrund des Vorhandenseins der Zielsequenzen für die ortsspezifische Rekombinase ein einfaches Ersetzen der ersten heterologen Expressionskontrollsequenz oder/und des ersten Amplifikationsgens durch eine zweite heterologe Expressionskontrollsequenz oder/und ein zweites Amplifikationsgen möglich ist.

Die Bezeichnung "ortsspezifische Rekombinase" gemäß vorliegender Erfindung umfaßt Proteine und Proteinkomplexe, die DNA-Umlagerungen an einer spezifischen DNA-Zielsequenz vermitteln, einschließlich ortsspezifischer Rekombinasen der Integrase- oder Resolvase-Invertase-Klassen (Stark et al., Trends Genet. 8 (1992), 432-439; Abremski und Hoess, Protein Engineering 5 (1992), 87-91; Khan et al., Nucleic Acids Res. 19 (1991), 851-860) und durch Intron-kodierte Endonukleasen vermittelte ortsspezifische Rekombination (Perrin et al., EMBO J. 12 (1993), 2939-2947). Bevorzugte Rekombinaseproteine werden ausgewählt aus der Gruppe bestehend aus der FLP-Rekombinase des 2 *µ* Episoms von Saccharomyces cerevisiae (z.B. Falco et al., Cell 29 (1982), 573-584; Cox, Proc. Natl. Acad. Sci. USA 80 (1983) 4223-4227; Konsolaki et al., New Biologist 4 (1992), 551-557), der Cre-Rekombinase des E.coli Phagen P1 (z.B. Sauer und Henderson (1989) supra), der R-Rekombinase aus dem Zygosaccharomyces rouxii Plasmid pSR1 (Matsuzaki et al., J. Bacteriol. 172 (1990), 610-618), der A-Rekombinase aus dem Kluyveromyces drososphilarium Plasmid pKD1 (Chen et al., Nucleic Acids Res. 14 (1986), 4471-4481), der A-Rekombinase aus dem Kluveromyces waltii-Plasmid pKW1 (Chen et al., J. Gen. Microbiol. 138 (1992), 337-345), einer Komponente des λ-Int-Rekombinationssystems (Landy, Annu Rev. Biochem. 5( (1989), 913-949) und einer Komponente des Gin-Rekombinationssystems des Phagen *µ* (Klippel et al., EMBO J. 12 (1993), 1047-1057). Darüber hinaus sind auch die im europäischen Patent EP-B-O 707 599 beschriebenen Fusionsproteine aus einer ortsspezifischen Rekombinase und einem nuklearen Rezeptor oder der ligandenbindenden Domäne davon geeignet. Besonders bevorzugt werden für das erfindungsgemäße Verfahren Zielsequenzen der Cre-Rekombinase, d.h. loxP-Sequenzen, verwendet.

Im Gegensatz zur rekombinanten Herstellung von Proteinen durch ortsunspezifische Integration heterologer Gene und den damit verbundenen Nachteilen werden mit dem erfindungsgemäßen Verfahren die Vorteile der ortsspezifischen endogenen Genaktivierung durch homologe Rekombination genützt. Durch eine vereinfachte Auswahl geeigneter Kombinationen von heterologen Expressionskontrollsequenzen und Amplifikationsgenen erhält man mit hoher Wahrscheinlichkeit optimierte Herstellungsklone mit stabilen Eigenschaften, welche die Herstellung eines Proteins ermöglichen, das in seiner Struktur und Aktivität weitgehend mit dem nativen Protein übereinstimmt.

Die Auswahl geeigneter homologer Sequenzen, die die heterologe Expressionskontrollsequenz, das Amplifikationsgen, das positive Selektionsmarkergen und die Rekombinase-Zielsequenzen flankieren, erfolgt beispielsweise gemäß den in WO90/11354 und WO91/09955 beschriebenen Methoden.

Darüber hinaus können in den homologen Sequenzen auch Modifikationen enthalten sein, die im exprimierten Protein zu Mutationen, wie beispielsweise Punktmutationen, Insertionen oder/und Deletionen einzelner Aminosäuren oder ganzer Aminosäureabschnitte führen.

Mit dem erfindungsgemäßen Verfahren wird es somit möglich in einem einzigen Verfahrensschritt nicht nur die Expressionshöhe einer endogenen Nukleinsäuresequenz zu verändern, sondern gleichzeitig eine Mutation in den kodierenden Bereich der endogenen Nukleinsäuresequenz einzuführen. Somit ist das erfindungsgemäße Verfahren besonders vorteilhaft bei der Herstellung von Proteinen für Arzneimittelanwendungen. Derartige Proteine sollen außer Mutationen zur Wirksamkeitsteigerung des Proteins keine weiteren Veränderungen im Vergleich zu nativen Proteinen aufweisen.

Erfindungsgemäß kann jede eurkaryontische Zelle verwendet werden, vorzugsweise wird eine Säugerzelle, besonders bevorzugt eine humane Zelle verwendet. Das erfindungsgemäße Verfahren kann mit nichtimmortalisierten Zellen, z.B. Fibroblasten, aber auch mit immortalisierten Zellen, z.B. Tumorzellinien, durchgeführt werden. Bevorzugt sind immortalisierte Zellen.

Die bei der Durchführung des erfindungsgemäßen Verfahrens verwendeten Lösungen und Medien werden vorzugsweise so ausgewählt, daß im jeweiligen Verfahrensschritt optimale Bedingungen vorliegen. Die Kultivierung der Zellen erfolgt mit Medien, die alle für ein ausreichendes Zellwachstum nötigen Stoffe enthalten und gegebenenfalls gepuffert sind. Vorzugsweise sind die Zellen in serumfreiem Medium kultivierbar. Besonders bevorzugt ist die verwendete Zelle eine Namalwa-, HT1080 oder HeLa S3 Zelle.

Das erfindungsgemäße Verfahren ermöglicht die Optimierung der Expression einer in der Zelle endogen vorliegenden Nukleinsäuresequenz, d.h. eines Zielgens durch Auswahl einer optimalen Expressionskontrollsequenz, eines optimalen Amplifikationsgens oder/und durch Auswahl einer optimalen Kombination von Expressionskontrollsequenz und Amplifikationsgen.

Als heterologe Expessionskontrollsequenz kann jede Nukleinsäuresequenz verwendet werden, die nach ihrer Integration in das Genom der Zelle die Expression des Zielgens beeinflußt. Dies umfaßt Nukleinsäuresequenzen, die direkte Wechselwirkungen mit Transkriptionskomponenten, wie beispielsweise Transkriptionsinitiationsfaktoren oder RNA-Polymerasen eingehen können und Nukleinsäuresequenzen, deren Einfluß auf die Transkription durch Wechselwirkungen mit Aktivatoren oder Repressoren vermittelt wird. Vorzugsweise umfaßt die heterologe Expressionskontrollsequenz einen Promotor/Enhancer, besonders bevorzugt virale Promotoren und am meisten bevorzugt einen CMV Promotor.

Die heterologe Expressionskontrollsequenz kann auch eine 3'-nichtkodierende Sequenz umfassen. 3'-nichtkodierende Sequenzen können stabilisierend oder destabilisierend auf eine mRNA wirken und erhöhen bzw. erniedrigen somit ihre Halbwertszeit. Durch Einführen einer eine mRNA stabilisierende Sequenz kann die Halbwertszeit einer mRNA und somit die Ausbeute des von ihr kodierten Proteins erhöht werden.

In einer bevorzugten Ausführungsform wird durch die homologe Rekombination eine endogene Expressionskontrollsequenz des Zielgens entfernt. Dies ist besonders vorteilhaft, wenn die endogene Sequenz eine Repressor bindende Sequenz umfaßt. Eine die Expression vermindernde Wirkung kann auch eine 3'-nichtkodierende Sequenz aufweisen, die destabilisierend auf die mRNA wirkt, wodurch die Menge an translatiertem Protein vermindert wird.

Weiterhin erlaubt das erfindungsgemäße Verfahren die Auswahl eines optimalen Amplifikationsgens. Das Amplifikationsgen wird vorzugsweise in einer exprimierbaren Form, d.h. in operativer Verknüpfung mit einem geeigneten Promotor eingesetzt und im Vektor so angeordnet, daß es sich nach der homologen Integration des Vektors in das Genom der eukaryontischen Zelle in räumlicher Nähe zum Zielgen befindet. Die Durchführung eines Amplifikationsschrittes führt zu einer Erhöhung der Anzahl von Kopien des Zielgens in der Zelle. Hierdurch kann eine weitere Expressionssteigerung der endogenen Nukleinsäuresequenz erreicht werden. Beispiele für geeignete Amplifikationsgene sind Dihydrofolatreduktase (DHFR), Adenosindeaminase, Ornithindecarboxylasebzw. Muteinedieser Gene. Vorzugsweise ist das Amplifikationsgen ein DHFR-Gen oder eine mutierte Form davon (Simonsen et al., Nucleic Acids Res. 1988, 16 (5): 2235-2246), insbesondere bei Zellen, die ein endogenes DHFR-Gen enthalten.

Als positiver Selektionsmarker kann jedes für eine eukaryontische Zelle geeignete Resistenzgen verwendet werden, welches zu einem selektierbaren Phänotyp führt, wie z.B. eine Antibiotikumresistenz. Vorzugsweise ist das positive Selektionsmarkergen ein Neomycin-, Kanamycin-, Geneticin- oder Hygromycin-Resistenzgen. Vorzugsweise wird das positive Selektionsmarkergen in exprimierbarer Form, d.h. in operativer Verknüpfung mit einem geeigneten Promotor verwendet.

Wird ein negatives Selektionsmarkergen verwendet, so wird üblicherweise zusätzlich zu dem positiven Selektionsschritt ein zweiter negativer Selektionsschritt durchgeführt. Dies bietet den Vorteil, daß nach Durchführung der Selektionsschritte die identifizierten Klone einen geringeren Anteil falsch-positiver Klone, d.h. zufällig in das Genom integrierte Vektoren, enthalten. Das negative Selektionsmarkergen ist vorzugsweise ein Thymidin-Kinase-Gen (TK) oder/und ein Hypoxanthin-Guanin-Phosphoribosyltransferase-Gen (HGPRT).

Aufgrund des Vorhandenseins der Zielsequenzen der ortsspezifischen Rekombinase können zwischen diesen Sequenzen lokalisierten Nukleinsäuresequenzen aus dem Genom der Zelle unter Verwendung der ortsspezifischen Rekombinase herausgeschnitten werden. Vorzugsweise wird die zwischen den Zielsequenzen lokalisierte Nukleinsäuresequenz aus dem Genom durch transiente Aktivierung der entsprechenden Rekombinase in der Zelle herausgeschnitten. Diese transiente Aktivierung der Rekombinase kann beispielsweise erfolgen durch
(a) Transfizieren der Zelle mit einem zweiten Vektor, umfassend eine für die Rekombinase kodierende Nukleinsäuresequenz operativ verbunden mit einer in dieser Zelle aktiven oder aktivierbaren Expressionskontrollsequenz und
(b) Kultivieren der so transfizierten Zelle unter Bedingungen, unter denen die Rekombinase exprimiert wird und aktiv ist und
(c) gegebenenfalls Gewinnen der Zelle.

Bei Verwendung von Rekombinase/Nuklearer Rezeptor-Fusionsproteinen kann die transiente Aktivierung der Zelle auch durch gesteuerte Zugabe des Liganden für den nuklearen Rezeptor erfolgen.

Nach Entfernen der zwischen den Zielsequenzen liegenden DNA kann die verbleibende Zielsequenz, z.B. die loxP-Sequenz, für weitere Verfahrensschritte genutzt werden.

In einer weiteren bevorzugten Ausführungsform ist das Verfahren dadurch gekennzeichnet, daß
(a) die Zelle transfiziert wird mit einem dritten Vektor, umfassend
   (i) mindestens eine Sequenz ausgewählt aus einer zweiten heterologen Expressionskontrollsequenz und einen zweiten Amptifikationsgen,
   (ii) ein positives Selektionsmarkergen, das sich vorzugsweise von dem positiven Selektionsmarkergen des ersten Vektors unterscheidet und
   (iii) mindestens zwei die Sequenzen (i) und (ii) flankierende Rekombinase-Zielsequenzen,
(b) die transfizierte Zelle unter Bedingungen kultiviert wird, unter denen eine Integration der von den Zielsequenzen flankierten Sequenz in die Zielsequenz im Genom der Zelle erfolgt,
(c) die gemäß Schritt (b) erhaltene Zelle gewonnen wird und
(d) gegebenenfalls die Schritte (a) bis (c) mindestens einmal mit jeweils variierenden Expressionskontrollsequenzen oder/und Amplifikatiansgenen wiederholt werden.

Mit dem erfindungsgemäßen Verfahren können somit viele Expressionskontrollsequenzen, Amplifikationsgene oder Kombinationen von Expressionskontrollsequenzen und Amplifikationsgenen einfach und schnell getestet werden. Die Durchführung einer zeit- und kostenaufwendigen ortsspezifischen Integration für jede einzelne heterologe Expressionskontrollsequenz bzw. jedes einzelne Amplifikationsgen zur Ermittlung eines optimalen Expressions/Amplifikationssystems für jedes einzelne Zielgen entfällt somit.

Das positive Selektionsmarkergen in einem dritten Vektor unterscheidet sich vorzugsweise von dem eines ersten Vektors, um das Selektionsverfahren zu vereinfachen und die Zahl der falsch-positiven Klone zu minimieren.

Die Rekombinase-Zielsequenzen im erfindungsgemäß verwendeten Vektor können mit natürlich vorkommenden Zielsequenzen übereinstimmen oder gegebenenfalls Mutationen aufweisen, welche die Wirksamkeit der ortsspezifischen Rekombination nicht beeinträchtigen.

Offenbart wird auch ein Vektor für die homologe Rekombination, insbesondere für die ortsspezifische Einführung von Rekombinase-Zielsequenzen in das Genom einer Zelle, umfassend
(i) eine heterologe Expressionskontrollsequenz und gegebenenfalls zusätzlich ein Amplifikationsgen,
(ii) ein positives Selektionsmarkergen,
(iii) mindestens zwei die Sequenzen (i) und (ii) flankierende Zielsequenzen für eine ortsspezifische Rekombinase,
(iv) die Sequenzen (i), (ii) und (iii) flankierende DNA-Sequenzen, die homolog zu einem Nukleinsäureabschnitt im Genom einer Zelle sind, um eine homologe Rekombination zu erlauben, und
(v) gegebenenfalls ein negatives Selektionsmarkergen.

Alle erfindungsgemäßen Vektoren enthalten weiterhin vorzugsweise die für eine Propagierung und Vermehrung in geeigneten Wirtszellen nötigen Sequenzelemente, wie beispielsweise Replikationsursprung, Selektionsmarkergene etc.

Noch ein weiterer Gegenstand der vorliegenden Erfindung ist eine eukaryontische Zelle, vorzugsweise eine humane Zelle, die erhältich ist durch ein wie oben beschriebenes Verfahren. Diese Zelle, z.B. eine humane Zelle, ist vorzugsweise dadurch gekennzeichnet ist, daß sie
(a) eine chromosomal lokalisierte heterologe Expressionskontrollsequenz und gegebenenfalls zusätzlich ein Amplifikationsgen in operativer Verknüpfung mit einem endogenen Gen enthält, wobei die heterologe Expressionskontrollsequenz die Expression des Zielgens in der Zelle erhöht, und wobei
(b) die chromosomal lokalisierte Sequenz flankiert ist von Rekombinase-Zielsequenzen.

Die Erfindung wird durch die nachfolgenden Beispiele, Figuren und das Sequenzprotokoll erläutert.

### Figurenbeschreibung

### Figur 1

(A) zeigt einen Vektor für die homologe Rekombination, der als erster Vektor verwendet wird. HR: homologe Sequenz, Seq 1: erste heterologe Expressionskontrollsequenz, R1: positives Selektionsmarkergen, loxP: loxP-Sequenz mit Orientierung,
(B) zeigt genomische Sequenzen
   (a) nach erfolgter homologer Rekombination,
   (b) nach durch eine Cre-Rekombinase katalysiertem Herausschneiden einer von loxP-Sequenzen flankierten Sequenz,
(C) zeigt einen Vektor für eine Cre-Rekombinase vermittelte Integration, der eine Sequenz zwischen loxP-Sequenzen angeordnet umfaßt,
   (c) zeigt genomische Sequenzen nach Integration eines zweiten Vektors an der loxP-Sequenz. R2: positives Selektionsmarkergen, welches sich gegebenenfalls von R1 unterscheidet, Seq 2: zweite heterologe Expressionskontrollsequenz.

### Figur 2

(A) zeigt einen Vektor für die homologe Rekombination HR: homologe Sequenz, R-box: positives und gegebenenfalls negatives Selektionsmarkergen, loxP: loxP-Sequenz mit Orientierung, HSV-tk: Herpes simplex-Thymidinkinase;
(B) zeigt einen Vektor für die homologe Rekombination mit einseitiger homologer Sequenz.

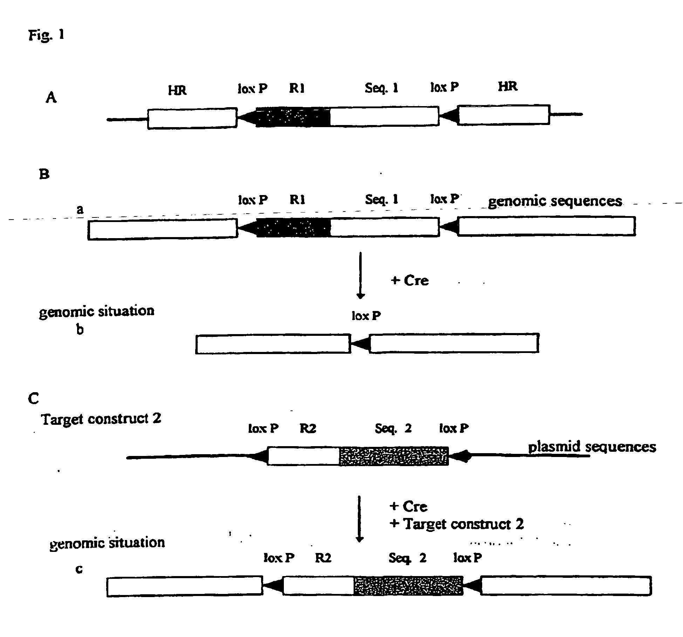

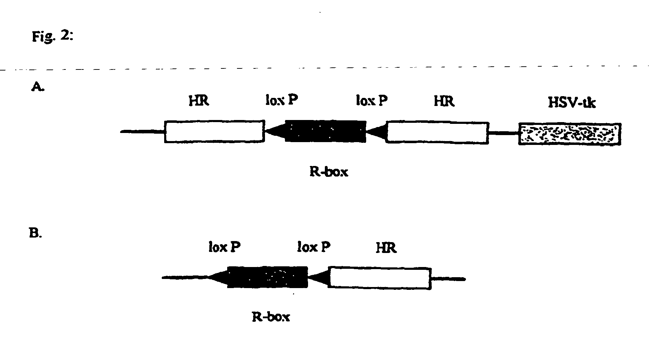

## Patentansprüche

1. Verfahren zum Verändern der Expression eines in einer eukaryontischen Zelle endogen vorliegenden Gens,
**dadurch gekennzeichnet,**
**dass**
(a) die Zelle transfiziert wird mit einem ersten Vektor, umfassend
(i) eine heterologe Expressionskontrollsequenz, um die Expression des Zielgens in der Zelle zu erhöhen, und gegebenenfalls zusätzlich ein Amplifikationsgen,
(ii) ein positives Selektionsmarkergen,
(iii) mindestens jeweils zwei die Sequenz (i) und (ii) flankierende Zielsequenzen für eine ortsspezifische Rekombinase,
(iv) die Sequenzen (i), (ii) und (iii) flankierende DNA-Sequenzen, die homolog zu einem Nukleinsäureabschnitt im Genom der Zelle sind, um eine homologe Rekombination zu erlauben,
(b) die transfizierte Zelle unter Bedingungen kultiviert wird, unter denen eine homologe Rekombination des Vektors erfolgt, und
(c) die gemäß Schritt (b) erhaltene Zelle gewonnen wird, die das endogene Gen in operativer Verknüpfung mit der heterologen Expressionskontrollsequenz aufweist, wobei diese Sequenzen flankiert sind von den Zielsequenzen fur die ortsspezifische Rekombinase.

2. Verfahren nach Anspruch 1,
**dadurch** gekennzeichent,
dass man als Rekombinase-Zielsequenzen loxP-Sequenzen verwendet.

3. Verfahren nach einem der vorhergehenden Ansprüche,
**dadurch gekennzeichnet**,
das der Vektor weiterhin ein negatives Selektionsmarkergen umfasst, welches außerhalb der homologen Sequenzen gemäß Anspruch 1 (a) (iv) angeordnet ist.

4. Verfahren nach einem der vorhergehenden Ansprüche,
**dadurch gekennzeichnet,**
**dass** die zwischen den Rekombinase-Zielsequenzen lokalisierte Nukleinsäuresequenz durch transiente Aktivierung einer die Zielsequenzen erkennenden ortsspezifischen Rekombinase aus dem Genom der Zelle herausgeschnitten wird.

5. Verfahren nach einem der Ansprüche 1 bis 4,
**dadurch gekennzeichnet,**
**dass** (a) die zwischen den Recombinase-Zielsequenzen lokalisierte Nukleinsäuresequenz durch transierte Aktivierung einer die Zielsequenzen erkennenden ortsspezifischen Rekombinase aus dem Genom der Zelle herausgeschnitten wird,
(b) die Zelle transfiziert wird mit einem weiteren Vektor, umfassend
(i) mindestens eine Sequenz ausgewählt aus einer zweiten heterologen Expressionskontrollsequenz und einem zweiten Amplifikationsgen,
(ii) ein positives Selektionsmarkergen und
(iii) mindestens zwei die Sequenzen (i) und (ii) flankierende Rekombinase-Zielsequenzen,
(c) die transfizierte Zelle unter Bedingungen kultiviert wird, unter denen eine Integration der von den Zielsequenzen flankierten Sequenz in die Zielsequenz im Genom der Zelle erfolgt,
(d) die gemäß Schritt (c) erhaltene Zelle gewonnen wird und
(e) gegebenenfalls die Schritte (b) bis (d) mindestens einmal mit jeweils variierenden Expressionskontrollsequenzen oder/und Amplifikationsgenen wiederholt werden.

6. Eukaryontische Zelle, vorzugsweise humane Zelle,
**dadurch gekennzeichnet,**
**dass** sie
(a) eine chromosomal lokalisierte heterologe Expressionskontrollsequenz und gegebenenfalls zusätzlich ein Amplifikationsgen in operativer Verknüpfung mit einem endogenen Gen enthält, wobei die heterologe Expressionkontrollsequenz die Expression des Zielgens in der Zelle erhöht, und wobei
(b) die chromosomal lokalisierte Sequenz flankiert ist von Rekombinase-Zielsequenzen.

## Claims

1. Process for changing the expression of a gene which is present endogenously in a eukaryotic cell,
**characterized in that**
(a) the cell is transfected with a first vector, comprising
(i) a heterologous expression control sequence in order to increase the expression of the target gene in the cell and optionally in addition an amplification gene,
(ii) a positive selection marker gene,
(iii) at least two target sequences for a site-specific recombinase which flank the sequences (i) and (II),
(iv) DNA sequences which flank the sequences (i), (ii) and (iii) and are homologous to a nucleic acid section in the genome of the cell in order to allow a homologous recombination,
(b) the transfected cell is cultured under conditions under which a homologous recombination of the vector takes place and
(c) the cell obtained according to step (b) which has the endogenous gene in operative linkage with the heterologous expression control sequence, where these sequences are flanked by the target sequences for the site-specific recombinase, is isolated.

2. Process according to claim 1,
**characterized in that**
lox-P sequences are used as recombinase target sequences.

3. Process according to one of the previous claims,
**characterized in that**
the vector additionally contains a negative selection marker gene which is located outside the homologous sequences according to claim 1 (a) (iv).

4. Process according to one of the previous claims,
**characterized in that**
the nucleic acid sequence located between the recombinase target sequences is cut out of the genome of the cell by transient activation of a site-specific recombinase that recognizes the target sequences.

5. Process according to one of the claims 1 to 4,
**characterized in that**
(a) the nucleic acid sequence that is located between the recombinase target sequences is cut out of the genome of the cell by transient activation of a site-specific recombinase that recognizes the target sequences,
(b) the cell is transfected with an additional vector, comprising
(i) at least one sequence selected from a second heterologous expression control sequence and a second amplification gene,
(ii) a positive selection marker gene, and
(iii) at least two recombinase target sequences that flank the sequences (i) and (ii),
(c) the transfected cell is cultured under conditions under which the sequence flanked by the target sequences is integrated into the target sequence in the genome of the cell,
(d) the cell obtained according to step (c) is isolated, and
(e) the steps (b) to (d) are optionally repeated at least once using in each case different expression control sequences or/and amplification genes.

6. Eukaryotic cell, preferably human cell,
**characterized in that** it
(a) contains a chromosomally located heterologous expression control sequence and optionally in addition an amplification gene in operative linkage with an endogenous gene, where the heterologous expression control sequence increases the expression of the target gene in the cell and wherein,
(b) the chromosomally located sequence is flanked by recombinase target sequences.

## Revendications

1. Procédé de modification de l'expression d'un gène se trouvant de manière endogène dans une cellule eucaryote, **caractérisé en ce que**
(a) on transfecte la cellule avec un premier vecteur comprenant
(i) une séquence de contrôle de l'expression hétérologue pour augmenter l'expression du gène cible dans la cellule, et éventuellement en plus un gène d'amplification,
(ii) un gène de marqueur de sélection positive,
(iii) au moins à chaque fois deux séquences cibles pour une recombinase spécifique d'un site, flanquant la séquence (i) et (ii),
(iv) des séquences d'ADN flanquant les séquences (i), (ii) et (iii), qui sont homologues d'un fragment d'acide nucléique du génome de la cellule, pour permettre une recombinaison homologue,
(b) on cultive la cellule transfectée dans des conditions dans lesquelles il se produit une recombinaison homologue du vecteur, et
(c) on récupère la cellule obtenue selon l'étape (b), qui présente le gène endogène en liaison opérationnelle avec la séquence de contrôle de l'expression hétérologue, ces séquences étant flanquées par les séquences cibles pour la recombinase spécifique d'un site.

2. Procédé selon la revendication 1, **caractérisé en ce que** l'on utilise des séquences loxP comme séquences cibles pour une recombinase.

3. Procédé selon l'une des revendications précédentes, **caractérisé en ce que** le vecteur comprend en outre un gène de marqueur de sélection négative qui est placé en-dehors des séquences homologues selon la revendication 1(a)(iv).

4. Procédé selon l'une des revendications précédentes, **caractérisé en ce que** la séquence d'acide nucléique localisée entre les séquences cibles pour la recombinase est excisée du génome de la cellule par activation temporaire d'une recombinase spécifique d'un site reconnaissant les séquences cibles.

5. Procédé selon l'une des revendications 1 à 4, **caractérisé en ce que**
(a) on excise du génome de la cellule la séquence d'acide nucléique localisée entre les séquences cibles pour une recombinase par activation temporaire d'une recombinase spécifique d'un site reconnaissant les séquences cibles,
(b) on transfecte la cellule avec un autre vecteur comprenant
(i) au moins une séquence choisie parmi une deuxième séquence de contrôle de l'expression hétérologue et un deuxième gène d'amplification,
(ii) un gène de marqueur de sélection positive,
(iii) au moins deux séquences cibles pour une recombinase, flanquant les séquences (i) et (ii),
(c) on cultive la cellule transfectée dans des conditions dans lesquelles il se produit une intégration de la séquence flanquée par les séquences cibles dans la séquence cible du génome de la cellule,
(d) on récupère la cellule obtenue selon l'étape (c) et
(e) on répète éventuellement au moins une fois les étapes (b) à (d) avec à chaque fois des séquences de contrôle de l'expression et/ou des gènes d'amplification qui varient.

6. Cellule eucaryote, de préférence une cellule humaine, **caractérisée en ce que**
(a) elle contient une séquence de contrôle de l'expression hétérologue localisée sur un chromosome et éventuellement en plus un gène d'amplification en liaison opérationnelle avec un gène endogène, la séquence de contrôle de l'expression hétérologue augmentant l'expression du gène cible dans la cellule, et
(b) la séquence localisée sur un chromosome est flanquée par des séquences cibles pour une recombinase.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Strasse 112-132
      (C) ORT: Mannheim
      (E) LAND: Deutschland
      (F) POSTLEITZAHL: D-68305
   (ii) BEZEICHNUNG DER ERFINDUNG: Optimierung von Zellen fuer die endogene Genaktivierung
   (iii) ANZAHL DER SEQUENZEN: 3
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 53 Basenpaare
      (B) ART: Nucleotid
      (C) STRANGFORM: beides
      (D) TOPOLOGIE: linear
   (xi) SEQUENZBESCHREIBUNG: SEQ ID NO: 1:
      CCTCTCCTCT AGGCCCGTGG GGCTGGCCCT GCACCGCCGA GCTTCCCGGG ATG
(2) ANGABEN ZU SEQ ID NO: 2:
   (i) SEQUENZKENNZEICHEN:
      (A) LÄNGE: 43 Basenpaare
      (B) ART: Nucleotid
      (C) STRANGFORM: beides
      (D) TOPOLOGIE: linear
   (xi) SEQUENZBESCHREIBUNG: SEQ ID NO: 2:
      CTACGTGCTG TCTCACACAG CCTGTCTGAC CTCTCGACCC TAC
(2) ANGABEN ZU SEQ ID NO: 3:
   (i) SEQUENZKENNZEICHEN:
      (A) LÄNGE: 34 Basenpaare
      (B) ART: Nucleotid
      (C) STRANGFORM: beides
      (D) TOPOLOGIE: linear
   (xi) SEQUENZBESCHREIBUNG: SEQ ID NO: 3:
      TATTGAAGCA TATTACATAC GATATGCTTC AATA